# EUROPEAN PATENT SPECIFICATION

(11) **EP 0 769 966 B1**
(45) Date of publication and mention of the grant of the patent: **02.06.2004**
(21) Application number: 95922233.2
(22) Date of filing: 05.06.1995
(51) Int. Cl.: A61K 47/48, A61K 51/04, A61P 35/00, A61P 31/00

(54) **BIOTIN COMPOUNDS FOR TARGETTING TUMORS AND SITES OF INFECTION**
BIOTINVERBINDUNGEN ZUM ZIELEN AUF TUMORE UND INFEKTIONSHERDE
COMPOSES DE BIOTINE POUR CIBLER DES TUMEURS ET DES FOYERS D'INFECTION

(30) Priority: 06.06.1994 US 254260; 24.06.1994 US 265516
(43) Date of publication of application: 02.05.1997
(73) Proprietor: THEJMDE Trust, Newton, Massachusetts 02159 (US)
(72) Inventor: ELMALEH, David R., Boston, MA 02110 (US); FISCHMAN, Alan J., Boston, MA 02114 (US); SHOUP, Timothy M., De Catur, GA 30033 (US); BABICH, John W., North Scituate, MA 02066 (US)
(74) Representative: Deans, Michael John Percy
(86) International application number: PCT/US1995/007184
(87) International publication number: WO 1995/033491

(56) References cited:
- EP-A- 0 451 810
- WO-A-90/12096
- WO-A-93/17714
- WO-A-93/25240
- WO-A-95/14493
- PROC. NATL. ACAD. SCI. U. S. A. (1976), 73(10), 3516-18 CODEN: PNASA6, 1976 HOFMANN, KLAUS ET AL 'An approach to the targeted attachment of peptides and proteins to solid supports'
- J. NUCL. MED. (1994), 35(10), 1685-90 CODEN: JNMEAQ;ISSN: 0161-5505, 1994 SHOUP, TIMOTHY M. ET AL 'Synthesis of fluorine-18-labeled biotin derivatives: Biodistribution and infection localization'
- CHEMICAL ABSTRACTS, vol. 113, no. 15, 8 October 1990 Columbus, Ohio, US; abstract no. 128783, ZHANG, ZHAOSONG ET AL 'Preparation of photobiotin-DNA probes and their use for detection of Plasmodium falciparum DNA' & NANJING YIXUEYUAN XUEBAO (1990), 10(2), 90-3 CODEN: NAYXEW;ISSN: 1000-5331, 1990
- ANGEWANDTE CHEMIE. INTERNATIONAL EDITION, vol. 31, no. 11, 1 November 1992 pages 1507-1509, XP 000325384 KOCH P ET AL '99MTC LABELED BIOTIN CONJUGATE IN A TUMOR "PRETARGETING" APPROACH WITH MONOCLONAL ANTIBODIES'
- JOURNAL OF BIOLOGICAL CHEMISTRY (MICROFILMS), vol. 263, no. 1, 5 January 1988 MD US, page 210-215 RUSSEL K. GARLICK ET AL. 'AVIDIN BINDING OF RADIOLABELED BIOTIN DERIVATIVES.'
- JOURNAL OF NUCLEAR MEDICINE, vol. 33, no. 5, May 1992 NEW YORK US, page 1026 T.M. SHOUP ET AL. 'THE EVALUATION OF F-18 LABELLED BIOTIN DERIVATIVES FOR TUMOR LOCALIZATION WITH PET.'

## Description

High contrast imaging of tumors can be achieved by allowing a nonradiolabeled antibody to localize and clear from the circulation prior to administration of a low molecular weight. radiolabeled moiety with high affinity for the pretargeted antibody (Paganelli, G. et al., *J. Nucl. Med. Comm. 12*:211-234 (1991); Green, NM *Biochem. J. 89*:585-91 (1963); Hnatowich DJ et al., *J. Nucl. Med. 28*:1294-1302 (1987)). One such method utilizes the high affinity of avidin, a cationic glycoprotein found in egg whites. for biotin, a naturally occuring vitamin. Avidin is capable of binding four biotin molecules and forming an avidin-biotin complex with a very high affinity.(Kd = 10⁻¹⁵M).

Two basic approaches for targeting tumors with avidin-biotin systems have been used in patients and animals. In the first method, avidin (or streptavidin)-conjugated antibodies are injected and days later, when antibody-tumor binding is maximized, a radioactive biotin derivative is injected to localize the tumor. Unfortunately, incomplete clearance of unbound antibody from the blood circulation can obscure visualization of the target site. In the second method, blood background is reduced by injecting biotinylated antibodies followed three days later by cold avidin. The resultant circulating biotinylated antibody-avidin complexes are sequestered from the blood by the liver. Radioactive biotin is then injected and binds to the antibody-biotin-avidin complexes already localized in the tumor. However, by employing "pretargetting" steps, both approaches for targeting tumors require that a subject be available to undergo multiple procedures over the course of a few days.

A study by Morrel et al., reported uptake of In-111 labeled IgG and human serum albumin (HSA) in an E. *coli* infected rat model. The accumulation of both labeled proteins was found to be sufficient to produce clear images of the infection site (Morrel, EM et al., *J. Nucl. Med. 30*:1538-1545 (1989).

Simple, rapid methods for more specifically targeting therapeutic or imaging agents to tumors and sites of infection in vivo are needed.

According to a first aspect of the invention, we provide use of a biotin compound in the manufacture of a medicament or diagnostic material, for use in a method of therapy or diagnosis involving targeting of said compound to a site in a patient without pre-administration of avidin or streptavidin, wherein the biotin compound is biotin (hexahydro-2-oxo-1H-thieno [3,4-d]-imidazoline-4-valeric acid); or a biotin amide analogue having structural formula I, below. 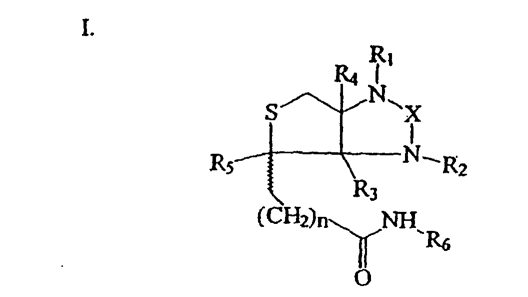 wherein,
- n=: 2-10;
- R₁-R₅=: H, acyl, alkyl, alkylene, alkenylene, alkynylene, alkenyl or alkynyl groups;
- R₆=: H, acyl, alkyl, alkylene, alkenylene, alkynylene, alkenyl or alkynyl groups; or a conjugated therapeutic agent or imaging agent; and
- X=: C=O, S=O or C=NH.

We describe hereinbelow biotin compounds that have a high specificity for target sites when administered to a subject in vivo. Preferred biotin compounds are biotin amide analogues and preferred target sites include tumours and sites of bacterial, viral or fungal infection. Particularly preferred biotin compounds show a target to non-target ratio of at least 5:1, are stable *in vivo* and substantially localize to target within 1 hour after administration. An especially preferred biotin amide analogue is [3aS-(3aα, 4β, 6aα)]-hexahydro-2-oxo-1H-thieno [3,4d]imidazole-4-(N-3-(1-fluoropropyl))pentanamide.

We describe pharmaceutical compositions comprised of a biotin compound. Preferred pharmaceutical compositions are biotin amide analogues having a therapeutic agent in the R₆ position. We describe new uses for biotin compounds in treating or preventing the establishment or growth of tumours or sites of infection (e.g. by pathogenic bacteria, viruses or fungi).

We describe biotin compounds comprising an imaging agent and uses for the compounds in detecting and/or monitoring tumours or sites of infection in a subject. In one embodiment, the biotin compound imaging agent is used to manufacture a diagnostic material to be administered in vivo and monitored using a means appropriate for the label. Preferred methods for detecting and/or monitoring a biotin compound imaging agent *in vivo* include Positron Emission Tomography (PET), and Single Photon Emission Computer Tomography (SPECT) and Magnetic Resonance Imaging (MRI).

The described biotin compounds and *in vivo* methods for targeting therapeutic or imaging agents to a tumour or site of infection do not require an initial pretargeting step. In addition, localization times are generally under 1 hour. Therefore, therapy or imaging can be accomplished in one short procedure. In addition, biotin compounds, which are natural small molecules that are rapidly cleared in vivo, are unlikely to have toxic side effects at the levels required for therapy or imaging.

Additional features and advantages will become more apparent from the following detailed description.

As used herein, the following terms and phrases shall have the meanings set forth below.

"Biotin compound" shall mean "biotin" (hexahydro-2-oxo-1H-thieno[3,4-d]-imidazoline-4-valeric acid); a 244 dalton vitamin, conjugated to an imaging agent or therapeutic agent; or a "biotin amide analogue", which is a compound having the following structural formula: 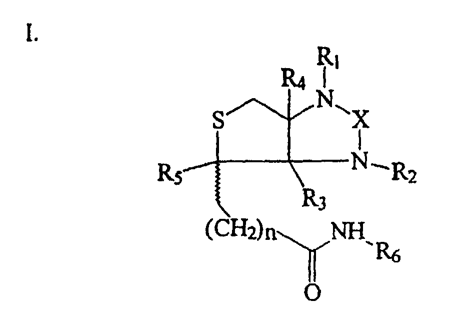 wherein,
- n=: 2-10;
- R₁-R₅=: H, acyl, alkyl, alkylene, alkenylene, alkynylene,alkenyl or alkynyl groups;
- R₆=: H, acyl, alkyl, alkylene, alkenylene, alkynylene,alkenyl or alkynyl groups; or a conjugated therapeutic agent or imaging agent; and
- X=: C=O, S=O or C=NH---.

Whilst not forming part of the present invention, compounds having the structural formula shown below are also disclosed herein for comparative purposes only. 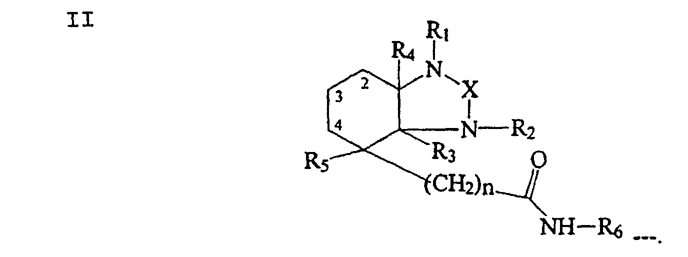 wherein,
- n=: 2-10;
- R₁-R₅=: H, acyl, alkyl, alkylene, alkenylene, alkynylene, alkenyl and alkynyl groups;
- R₆=: H, acyl, alkyl, alkylene, alkenylene, alkynylene, alkenyl and alkynyl groups; or a conjugated therapeutic agent or imaging agent
- X=: C=O, S=O or C=NH; and
- positions 2, 3, or 4 =: O, S, or N

The biotin amide analogs include racemic compounds and all possible enantiomers. The alkylene, alkenylene, alkynylene, alkenyl and alkynyl groups (hereinafter hydrocarbon groups) comprising the R groups can be either straight or branched chains, saturated or unsaturated. Unsaturated groups may have a single site of unsaturation or a plurality of unsaturated sites. The hydrocarbon groups preferably have up to about ten carbons, more preferably up to about six carbons, and most preferably up to about three carbons. A hydrocarbon group having three carbon atoms or less is considered to be a lower hydrocarbon group. For example, an alkyl group having three carbon atoms or less is a lower alkyl. Examples of lower hydrocarbon groups which may be used in the present invention include methyl, methylene, ethyl, ethylene, ethenyl, ethenylene, ethynl, ethynylene, propyl, propylene, propenyl, propenylene, propynyl, and propynylene. Examples of higher hydrocarbon groups (from four to about ten carbons) include butyl, t-butyl, butenyl, butenylene, and butynyl, butynylene, nonyl, nonylene, nonenyl, nonenylene, nonynyl, and nonynylene.

The alkyl or alkylene groups may be substituted with one or more oxygen or halogen atom to form alkoxy, haloalkyl, alkoxyene, and haloalkylene groups. The alkoxy and haloalkyl groups also may be straight or branched chain and preferably are made up of up to about ten atoms (including carbon, oxygen or halogen), preferably up to about six atoms, and most preferably up to about three atoms. The term halogen is art-recognized and includes chlorine, fluorine, bromine, astatine and iodine. Examples of substituted hydrocarbon groups which are useful within this invention are similar to hydrocarbon groups set forth above except for the incorporation of oxygen(s) or halogen(s) into the groups. Substitutions, which result in more lipophilic biotin compounds are preferred for targetting of tumors and sites of infection in the brain and spinal cord.

Especially preferred biotin compounds exhibit a high target to non-target ratio when administered in vivo. Preferably the ratio is ≥ 5:1. A biotin compound can be glycosylated with a sugar moiety (e.g. glucose, fucose, galactose, mannose) that is recognized by a target specific receptor to further increase the target specificity of a compound. For example, biotin compounds can be glycosylated with mannose residues (e.g. attached as C-glycosides to a free nitrogen) to yield biotin compounds having higher affinity binding to tumors expressing mannose receptors (e.g. glioblastomas and gangliocytomas); and bacteria, which are also known to express mannose receptors (Bertozzi, CR and MD Bednarski *Carbohydrate Research 223*:243 (1992); *J. Am. Chem. Soc. 114*:2242,5543 (1992)) as well as potentially other infectious agents.
"conjugated" shall mean ionically or covalently attached (e.g.via a crosslinking agent).

An "imaging agent" shall mean a composition capable of generating a detectable image upon binding with a target and shall include radionuclides (e.g.In-111, Tc-99m, I-123, I-125 F-18, Ga-67, Ga-68, and for Positron Emission Tomography (PET) and Single Photon Emission Tomography (SPECT), unpair spin atoms and free radicals (e.g. Fe, lanthides and Gd) and contrast agents (e.g. chelated (DTPA) manganese) for Magnetic Resonance Imaging (MRI).

A "therapeutic agent" shall mean an agent capable of having a biological effect on a host. Preferred therapeutic agents are capable of preventing the establishment or growth (systemic or local) of a tumor or infection. Examples include drugs (e.g. antibiotics, anti-virals, antifungals), toxins (e.g. ricin) , radionuclides (e.g. I-131, Re-186, Re-188, Y-90, Bi-212, At-211, Sr-89, Ho- 166, Sm-153, Cu-67 and Cu-64), hormone antagonists (e.g. tamoxifen), heavy metal complexes (e.g. cisplatin), oligonucleotides (e.g. antisense oligonucleotides that bind to a target nucleic acid sequence (e.g. mRNA sequence)), chemotherapeutic nucleotides, peptides, non-specific (non-antibody) proteins (e.g. sugar oligomers), boron containing compound (e.g. carborane), photodynamic agents (e.g. rhodamine 123), enediynes (e.g. calicheamicins, esperamicins, dynemicin, neocarzinostatin chromophore, and kedarcidin chromophore) and transcription based pharmaceuticals. In a preferred embodiment for treating or preventing the establishment or growth of a tumor, the therapeutic agent is a radionuclide, toxin, hormone antagonist, heavy metal complex, oligonucleotide, chemotherapeutic nucleotide, peptide, non-specific (non-antibody) protein, a boron compound or an enediyne. In a preferred embodiment for treating or preventing the establishment or growth of a bacterial infection, the therapeutic agent is an antibiotic, radionuclide or oligonucleotide. In a preferred embodiment for treating or preventing the establishment or growth of a viral infection, the therapeutic agent is an antiviral compound, radionuclide or oligonucleotide. In a preferred embodiment for treating or preventing the establishment or growth of a fungal infection, the therapeutic agent is an antifungal compound radionuclide or oligonucleotide.

A "target" shall mean an in vivo site to which biotin compounds bind. A preferred target is a tumor (e.g., tumors of the brain, lung (small cell and non-small cell), ovary, prostate, breast and colon as well as other carcinomas and sarcomas). Another preferred target is a site of infection (e.g. by bacteria, viruses (e.g. HIV, herpes, hepatitis) and pathogenic fungi *(Candida sp. ).* Particularly preferred target infectious organisms are those that are drug resistant (e.g. *Enterobacteriaceae, Enterococcus, Haemophilus influenza, Mycobacterium tuberculosis, Neisseria, gonorrhoeae, Plasmodium falciparum, Pseudomonas aeruginosa, Shigella dysenteriae, Staphylococcus aureus, Streptococcus pneumoniae*). The localization of biotin at a site of infection, may be related to that fact that it is an essential nutrient of many bacteria, viruses and fungi.

"Subject" shall mean a human or animal (e.g. rat, mouse, cow, pig, horse, sheep, monkey, cat, dog, goat etc)

### Method for Making Biotin Compounds

A variety of coupling or crosslinking agents such as protein A, carboiimide, dimaleimide, dithio-bis-nitrobenzoic acid (DTNB), N-succinimidyl-S-acetyl-thioacetate (SATA), and N-succinimidyl-3-(2-pyridyldithio) propionate (SPDP), 6-hydrazinonicotimide (HYNIC), N₃S and N₂S₂ can be used in well-known procedures to synthesize biotin amide analogs or biotin compounds. For example, biotin can be conjugated via DTPA using the bicyclic anhydride method of Hnatowich et al *Int. J. Appl. Radiat. Isotop. 33:* 327 (1982).

In addition sulfosuccinimidyl 6-(biotinamido) hexanoate (NHS-LC-biotin (which can be purchased from Pierce Chemical Co. Rockford, IL), "biocytin", a lysine conjugate of biotin, can be useful for making biotin compounds due to the availability of a primary amine. In addition, corresponding biotin acid chloride or acid precursors can be coupled with an aminoderivative of the therapeutic agent by known methods (See e.g. methods described in Examples 1 and 2).

Synthesized biotin compounds and biotin amide analogs can be characterized using standard methods of high field NMR spectra as well as IR, MS and optical rotation. Elemental analysis, TLC and/or HPLC can be used as a measure of purity. A purity of >98% is preferred. TLC and/or HPLC can also be used to characterize more lipophilic compounds.

Once prepared, candidate biotin derivatives can be screened for ability to bind avidin e.g. as described in the attached Example 3); for in vivo binding to sites of infection (e.g. as described in Example 4) or in vitro or in vivo binding to tumors. In addition, stability can be tested by administering the compound to a subject, obtaining blood samples at various time periods (e.g. 30 min, 1 hour, 24 hours) and analyzing the blood samples for the biotin compound and/or metabolites.

Two ¹⁸F biotin derivatives: an amide containing derivative (biotin 1) and an alkyl containing derivative (biotin 2) were prepared as described in Examples 1 and 2. Biotin 1 was easily synthesized from the sulfonate ester in good yield with high specific activity. Biotin 1 retains the basic structure of biotin. Biotin 2 was prepared to elucidate the role of the side chain on biotin binding to avidin and infection sites.

The two biotin derivatives were evaluated for binding to avidin in the presence of varying concentrations of unlabeled d-biotin as described in Example 3. Biotin 1 was found to bind to avidin and could be displaced by cold d-biotin. A scatchard transformation displacement curve yielded a Kd of 3.12 x 10 ⁻¹⁴ M with a Bmax of 5.57 x 10 ⁻¹⁶ M. In contrast, compound 2 showed no specific binding to avidin.

Both compounds were then evaluated for localization at sites of infection in *E. coli* infected rats as described in Example 4. Half of the infected rats were treated with avidin 24 hours prior to i.v. injection of the ¹⁸F-labeled biotin analogs. Biotin 1, without avidin pretreatment, showed a high selectivity (6.08 ± 1.12) for infected compared to normal muscle at 60 minutes post injection. This ratio increased only slightly by administration of avidin 24 hr prior to injection of ¹⁸F-labeled biotin 1 (6.39± 0.96).

In contrast, the biodistribution of biotin 2 indicated more binding to normal muscle with an infection/normal muscle ratio of 0.58 ± 0.07. This lack of selectivity illustrates the importance of the side-chain amide group in infection localization. Some defluorination of both biotin 1 and biotin 2 occured in vivo as evidenced by increased ¹⁸F bone uptake after 60 min.: 2.94 ± 0.37 and 1.17 ± 0.21 %IG/g±s.d.; respectively.

### Use of Biotin Compounds as Therapeutics

For use in therapy, an effective amount of an appropriate biotin compound can be administered to a subject by any mode, which allows the compound to be taken up by the appropriate target. Preferred routes of administration include oral and transdermal (e.g. via a patch). Examples of other routes of administration include injection (subcutaneous, intravenous, parenteral, intraperitoneal, intrathecal, etc.). The injection can be in a bolus or a continuous infusion. Depending on the route of administration, the biotin compound,therapeutic agent can be coated with or disposed in a selected material (e.g. positively or negatively charged liposomes), to protect it from natural conditions which may detrimentally effect its ability to perform its intended function, increase its in vivo availability or increase its uptake by a specific organ.

A biotin compound therapeutic agent can be administered alone, or in conjunction with a pharmaceutically acceptable carrier. As used herein, the phrase "pharmaceutically acceptable carrier" is intended to include substances that can be coadministered with a biotin compound therapeutic agent and allows the compound to perform its intended function. Examples of such carriers include solutions, solvents, dispersion media, delay agents, emulsions and the like. The use of such media for pharmaceutically active substances are well known in the art. Any other conventional carrier suitable for use with the biotin compounds also falls within the scope of the present invention.

The language "effective amount" of a biotin compound therapeutic agent refers to that amount necessary or sufficient to eliminate, reduce or maintain (e.g prevent the spread of) an infection, tumor or other target. The effective amount can vary depending on such factors as the disease or condition being treated, the particular biotin compound being administered, the size of the subject, or the severity of the disease or condition. One of ordinary skill in the art can empirically determine the effective amount of a particular compound without necessitating undue experimentation.

A preferred dose for treating or preventing a tumor or site of infection is in the range of 5 µg- 100mg. However, the exact dose depends to a great extent on the toxicity of the therapeutic agent being administered. For example, a subject can not withstand more than a milligram dose of bleomycin. In addition, certain chemotherapeutic peptides cause hemophilia and other blood disorders when given to a subject in microgram amounts. However, the selective targeting of a therapeutic agent by the instant biotin compounds decreases their otherwise toxic effects on normal body cells.

### Use of Biotin Compounds as Imaging Agents

Biotin compounds can be labeled with a variety of imaging agents, which are known in the art and which will depend to some extent on the means used to detect or monitor the compound in vivo or in vitro. Preferred imaging agents for performing positron emission tomography (PET), and single photon emission computer tomography (SPECT) include F-18, Tc-99m, and I-123. Preferred imaging agents for magnetic resonance imaging (MRI) include an appropriate atom with unpaired spin electrons or a free radical. An imaging agent can be complexed with a biotin compound by a variety of techniques that are well-known in the art. In a preferred embodiment, the imaging agent is attached via the amine to a HYNIC, DTPA or other chelating agent.

Biotin compounds that have been labeled with an appropriate imaging agent can be added to a particular tumor cell line or bacterial, viral or fungal infected tissue culture to test the binding affinity of a particular candidate biotin compound therapeutic.

Labeled biotin compounds can also be injected into an appropriate subject (e.g. monkey, dog, pig, cow) and its binding with tumors or sites of infection in vivo (e.g. as described in the following Example 4).

The present invention is further illustrated by the following examples which should not be construed as limiting in any way. The contents of all cited references (including literature references, issued patents, published patent applications, and co-pending patent applications) cited throughout this application are hereby expressly incorporated by reference.

### Example 1: Synthesis of Two F¹⁸-Labeled Biotin Analogs

Analysis for carbon and hydrogen was performed by Galbraith Laboratories (Knoxville, TN). Melting points were measured with a Fisher-Johns apparatus and are uncorrected. Proton NMR spectra (d₆-DMSO) were acquired with a Bruker AM 500 instrument; the chemical shifts are reported in parts per million (delta) down field from tetramethylsilane. All reagents were purchased from Aldrich Chemical Co., (Milwaukee, WI) and were used without further purification. Acetonitrile ws purchased from Pierce (Rockford, IL) and used as shipped.

Thin layer chromatography was performed on silica gel Gf 250 plates (Analtech, Inc., Newark, DE). Chromatograms of the radiolabeled compounds were counted with a Bioscan System 200 (Washington, D.C.). High pressure liquid chromatography was performed on a Resolvex C18 column (4.6 mm x 25 cm, Fisher Scientific Co.) at a flow rate of 1 ml/min using water/acetonitrile (60:40) solvent system. Separation was monitored with a U.V. detector (λ=185 nm) and a NaI (Tl) radioactivity detector.

### Synthesis of [3aS-(3aα, 4β, 6aα)]-hexahydro-2-oxo-1H-thieno [3, 4d]imidazole-4-(N-3-(1-fluoropropyl))pentanamide (Biotin 1)

(+)Biotin (1g, 4.09 mmol) was added to 20 ml of a stirred 50/50 mixture of thionyl chloride and benzene and the mixture was refluxed under N₂ for 2 h. Excess thionyl chloride was removed by distillation and the residue was cooled to room temperature under N₂, dissolved in dry THF (10 ml) and added to a stirred solution of 3-amino-1-propanol (1 g) in THF (20 ml) at 25°C. The solvent was removed by roto-evaporation, and excess amino propanol was removed at 120°C under vacuum (1 torr). The crude material was purified by chromatography on a silica gel column (30 g) eluted with CH₂Cl₂/MeOH/HCO₂H (20:80:0.5). Crystallization from MeOH gave 0.80g (65%) of product. This product showed a single spot (R_{f}=0.45) on TLC with CH₂Cl₂/MeOH/HCO₂H (20:80:0.5); mp=130-132°C. ¹H NMR (d₆-DMSO) d2.05 (t,J=6.2 Hz, 2H, CH₂-O), 3.2-3.5 (m, 6H, N-CH₂-), 6.35 (s, 1H, O=CNH-ring), 6.40 (s, 1H, O=CNH-ring), 7.75 (t, J=5.6 Hz, 1H, O=CNH). Anal. Calcd. for C₁₃H₂₃N₃O₃S: C, 51.80, H, 7.70. Found: C, 51.69; H, 7.51.

The product (100 mg, 0.33 mmol) was dissolved in 3 ml of dry pyridine by heating and stirring under N₂. The solution was cooled to room temperature, and 0.7 mL of a 0.87 M solution of methansulfonyl chloride in CH₂Cl₂ was added. After 30 min., the mixture was chromatographed on silica gel using acetone/acetonitrile (90:10). The product (86 mg, 69%) showed a single spot on TLC (R_{f}=0.49) with MeOH/CH₂Cl₂/HCO₂H (10:90:0.5) that was visulaized with MoO₃•H₃PO₄.

A 10-ml reactivial containing KF (48 mg), Kryptofix (300 mg), biotin mesylate (200 mg), and 7 ml of acetonitrile was heated at 110°C for 30 min. The solvent was roto-evaporated and the residue was chromatographed on silica gel (25 g) using CH₂Cl₂/MeOH/HCO₂H (85:15:0.5). This product, [3aS-(3aα, 4β, 6aα)]-hexahydro-2-oxo-1H-thieno [3,4d]imidazole- 4-(N-3-(1-fluoropropyl))pentanamide (Biotin 1), showed a single spot (R_{f}=0.38) by TLC with the same solvent. ¹H NMR (d₆-DMSO) d3.2-3.5 (m, 6H, N-CH₂-), 4.4 (dt, J=52, 6.5 Hz, 2H, CH₂-F), 6.35 (s, 1H, O=CNH-ring), 6.40 (s, 1H, O=CNH-ring), 7.75 (t, J=5.6 Hz, 1H, O=CNH). Anal. Calcd. for C₁₃H₂₂N₃O₂SF: C, 54.90, H, 7.80. Found: C, 52.19; H, 8.13.

### Synthesis of [3aS-(3aα, 4β, 6aα)]-tetrahydro-4-(5-(1-[¹⁸F]fluoro-pentyl)-1H-thieno[3,4-d]imidazol-2(3H)-one(Biotin 2) (for comparative purposes only)

To a stirred suspension of lithium aluminum hydride (LAH) (1.0g, 25.8 mmol) in anhydrous ether (125 ml), a hot pyridine (25 ml) solution of (+)biotin (1.0g, 4.01 mmol) was added dropwise. After addition, the reaction mixture was stirred at room temperature for 30 min. and then heated to reflux for an additional 30 min. The excess LAH was then destroyed by careful dropwise addition of water. Water and organic solvents were removed by roto-evaporation. The residue was acidified (pH 2) with 6 N HCl and the water was removed by roto-evaporation. The residue was extracted with chloroform (5 x 20 mL) and the choroform layer evaporated *in vacuo* to give a white solid. Recrystallization from methanol gave 0.44 g (50%) of the reduced product; mp 164-167°C (reference 11, mp 164-169°C). ¹H NMR (d₆-DMSO) d 4.35 (t, J=6 Hz, 2H, O-CH₂-), 6.35 (s, 1H, O=CNH-ring), 6.40 (s, 1H, O=CNH-ring).

To a solution of (+)Biotinol (100 mg, 0.43 mmol) in pyridine (3 ml) was added 0.6 mL of a 0.87 M solution of thionyl chloride in CH₂Cl₂. The mixture was stirred for 30 min. followed by chromatography on silica gel using acetone/acetonitrile 90:10. The product (96 mg, 75%) showed a single spot on TLC (R_{f}=0.42) with EtOAc/CH₂Cl₂/acetone/HCO₂H (10:80:10:0.5) that was visualized with MoO₃•H₃PO₄.

Biotin 2 was prepared from biotinol mesylate in the same manner as described for [3aS-(3aα, 4β, 6aα)]-hexahydro-2-oxo-1H-thieno [3,4d]imidazole 4-(N-3-(1-fluoropropyl))pentanamide above. [3aS-(3aα, 4β, 6aα)]-tetrahydro-4-(5-(1-[¹⁸F]fluoropentyl)-1H-thieno[3,4-d]imidazol-2(3H)-one was purified on silica gel using EtOAc/CH₂Cl₂/acetone/HCO₂H (10:80:10:0.5) (Rf=0.48); mp 110-112°C. ¹H NMR (d₆-DMSO) d 4.6 (dt, J=55, 6.5 Hz, F-CH₂-), 6.35 (s, 1H, O=CNH-ring), 6.40 (s, 1H, O=CNH-ring). Anal. Calcd. for C₁₀H₁₆N₂OSF: C, 56.31, H, 7.57. Found: C, 56.59; H, 7.79.

### Preparation of ¹⁸F-Labeled Biotin 1 and Biotin 2

^{**18**}**F-Labeled Biotin 1.** [¹⁸F] Fluoride was produced with a cyclotron by the ¹⁸O(p,n) ¹⁸F nuclear reaction on ¹⁸O-enriched water in a silver plated target at 17 MeV, 20mA-hr. (Scanditronix, Sweden, MC17F) (Kilbourn MR, Jerabek PA, Welch MJ. An improved [¹⁸O]water target for [¹⁸F]fluoride production. *Int J Appl Radiat Isot 36*:327-328 (1985).

A 5-ml reactivial containing ¹⁸F in H₂¹⁸O (50 mCi, 1 mL), Kryptofix (3 mg), and K₂CO₃ (1 mg) ws evaporated to dryness at 100°C under N₂. The contents were dried by addition of acetonitrile (4 x 2 mL) with heating at 110°C under a stream of N₂. Biotin mesylate (2 mg) in acetonitrile (1 mL) was added and the vial was sealed and heated at 110°C for 10 min. Solvent was removed and the labelled product purified by chromatography on a short column of silica gel (10 g) using MeOH/CH₂Cl₂/HCO₂H (20:80:0.5). The time required for synthesis and purification was 90 min. (from the end of bombardment ) and the radiochemical yield was 3.4 to 5.9 mCi (12-21%, EOS). HPLC analysis of the final product showed a single radioactive peak with a retention time (Rₜ) 2.8 min. corresponding to that of the [¹⁹F]fluoro-biotin. The chemical purity of [3aS-(3aα, 4β, 6aα)]-hexahydro-2-oxo-1H-thieno [3, 4-d]imidazole-4-(nnN-3-(1-[¹⁸F fluoropropyl))pentanamide was >8%.

^{**18**}**F-Labeled Biotin 2.** ¹⁸F-labeled Biotin 2 was prepared in the same manner as ¹⁸F-labeled Biotin 1. ¹⁸F-labeled Biotin 2 was purified on silica gel (10 g) using EtOAc/CH₂Cl₂/acetone/HCO₂H (10:80:10:0.5). The radiochemical yield was 5.6 mCi (20%, EOS) and radiochemical purity was determined to be greater than 98%. The chemical purity of 3aS-(3aα, 4β, 6aα)]-tetrahydro-4-(5-(1-[¹⁸F] fluoropentyl)-1H-thieno-[3, 4-d]imidazol-2(3H)-one was >98% by HPLC.

### Example 2: Competitive Binding of F-18 Labeled Biotin 1 and Biotin 2 to Avidin vs. d-Biotin

A preliminary measure of the binding characteristics of the fluoro analogs of biotin were performed. The binding of these novel F-18 labeled biotin analogs to avidin was evaluated by measuring the binding of F-18 labeled biotin analogs to avidin in the presence of varying concentrations of unlabeled d-biotin. Serial dilutions of d-biotin were prepared in phopshate buffered saline pH 7.4 (PBS). A fixed quantity of the no-carrier added F-18 labeled biotin 1 or 2 (10- 100µCi) were mixed with each d-biotin dilution so that the range of concentrations of cold biotin were 3 orders of magnitude greater and less than the concentration of avidin (10-¹⁶M). A 200 µl aliquot of the F-18 biotin/cold biotin solution was added to 200 µl of avidin in PBS. The solution was vortexed and allowed to incubate for 1 hour after which time a 5 µl aliquot of the solution was analysed by instant thin layer chromatography (Gelman ILTC-SG) using 0.1 M acetate buffer pH 6.0. In this chromatographic system the F-18 biotin analogs bound to avidin origin while the unbound F-18-biotin analogs move to the solvent front. Percent binding of F-18 biotin analog was calculated as [(counts at origin/total counts)* 100]. For each F-18-biotin analog potential impurities remaining at the origin were determined and the results of binding corrected accordingly.

### Example 3: Biodistribution Studies in Rats

A clinical isolate of *E. Coli* was stored at -70°C in a freezing media containing 20% glycerol and 80% dextrose phosphate until use. Aliquots of bacteria were defrosted and colony counts performed on serial dilutions grown overnight on BBL Brucella agar plates. Based on the colony count, freshly thawed bacteria suspensions were washed and diluted with saline to a final concentration of 8 x 10¹⁰ organisms/ml. Aliquots (0.1 ml) of bacterial suspensions (8 x 10⁹ organisms/ml) were injected into the right thigh muscle of 48 male Sprague-Dawley rats (125-150 g, Charles River Breeding Laboratories, Burlington, MD). Twenty-four rats were used for each labeled biotin compound. Twenty-four hours after injection, swelling in the right thigh readily appeared, and 24 rats each received 1.67 mg of avidin in saline via the tail vein. Forty-eight hours after bacterial inoculations, 80 to 150 mCi of ¹⁸F-biotin 1 or 2 was administered. Rats were sacrificed at 5 and 60 min. post-injection, six rats per time point. Blood samples were obtained by cardiac puncture. Syringes were weighed before and after injection to determine the volume delivered. The activity per unit volume was obtained from standards. A total of eleven different tissues were excised, weighed, and counted. These tissues included blood; bone; lung; liver; adrenal; spleen; kidneys; heart; muscle (thigh); teste; and infected muscle (thigh). The excised tissues were blotted, weighed, and counted in a scintillation well counter. The raw counts were decay corrected. The results were expressed as percent injected dose per gram, and infection-to-normal muscle ratios (mean ± s.d.). The biodistribution results were evaluated by analysis of variance (one-way ANOVA). The effect of avidin pretretment on biodistribution was evaluated by student t-test.

Table 1 shows the biodistribution of ¹⁸F-labeled biotin 1 at 5 and 60 min. after injection in rats with and without avidin pretreatment (24 hr). Analysis of variance demonstrated a significant main effects of organs (P<0.001) at 5 and 60 min. on biotin 1 accumulation. At 5 min. accumulation of 1 in kidney was greater than in all other organs (4.5-fold higher than blood) (P<0.001). Accumulation of 1 in liver and spleen were greater than normal muscle (P<0.02). Sufficient clearance of radioactivity had occurred within 60 min. in all other organs relative to infection except for bone (P<0.001). At 60 min., the selectivity for infected compared to normal muscle was 6.08± 1.12 (Table 2); accumulation in infected muscle was greater than kidney (P<0.05), blood (P<0.01), spleen (P<0.01), liver (P<0.001), and normal muscle (P<0.001). The distribution of biotin 1 in all organs did not change significantly with avidin-pretreatment at 5 min. At 60 min. with pretreatment, accumulation in bone (P<0.01) was less than without avidin-pretreatment. Compared to untreated rats, accumulation of 1 in avidin-treated rats was greater in kidney (P<0.02), blood (P<0.05), and normal muscle (P<0.002).

**Table 1.**

| **Tissue distribution of** ^{**18**}**F-labeled biotin 1 in *E. coli* infected rats** | | | | |
|---|---|---|---|---|
| Tissue | 5 Min. | Without Avidin 60 Min. | 5 Min. | With Avidin 60 Min. |
| Blood | 1.07±0.31 | 0.14±0.02 | 1.13±0.11 | 0.19±0.04 |
| Heart | 1.00±0.34 | 0.14±0.02 | 0.99±0.12 | 0.09±0.01 |
| Lung | 1.21±0.13 | 0.11±0.02 | 1.07±0.21 | 0.13±0.02 |
| Liver | 1.39±0.16 | 0.13±0.03 | 1.29±0.17 | 0.16±0.02 |
| Spleen | 1.40±0.30 | 0.14±0.03 | 1.52±0.32 | 0.19±0.0 |
| Kidney | 4.78±0.80 | 0.23±0.05 | 4.01±0.77 | 0.32±0.04 |
| Adrenal | 1.00±0.20 | 0.09±0.02 | 0.85±0.13 | 0.11±0.02 |
| Teste | 0.57±0.02 | 0.16±0.03 | 0.50±0.11 | 0.19±0.04 |
| Norm mus | 0.83±0.09 | 0.078±.02 | 0.82±0.12 | 0.10±0.02 |
| Inf mus | 1.22±0.36 | 0.43±0.10 | 0.99±0.11 | 0.65±0.14 |
| Bone | 1.10±0.16 | 2.94±0.37 | 1.19±0.23 | 2.29±0.14 |

**Table 2.**

| **Infected to normal tissue ratios of** ^{**18**}**F-labeled biotin 1 in *E. coli* infected rats.** | | | | |
|---|---|---|---|---|
| Tissue | Without Avidin 5 Min. | 60 Min. | With Avidin 5 Min. | 60 Min. |
| Blood | 1.30±0.86 | 3.09±0.54 | 0.90±0.14 | 3.75±0.75 |
| Heart | 1.21±0.26 | 6.21±0.57 | 1.08±0.18 | 7.59±1.34 |
| Lung | 1.00±0.23 | 4.21±0.69 | 1.06±0.30 | 4.83±0.91 |
| Liver | 0.89±0.25 | 3.48±0.67 | 0.83±0.16 | 4.05±0.65 |
| Spleen | 0.94±0.49 | 2.98±0.30 | 0.64±0.11 | 3.34±0.72 |
| Kidney | 0.26±0.05 | 1.96±0.24 | 0.28±0.06 | 2.04±0.35 |
| Adrenal | 1.06±0.24 | 5.20±1.20 | 1.20±0.22 | 5.74±1.14 |
| Teste | 2.14±0.60 | 2.87±0.50 | 2.00±0.37 | 3.35±0.56 |
| Norm mus | 1.54±0.69 | 6.08±1.12 | 1.24±0.20 | 6.39±0.96 |
| bone | 1.06±0.33 | 0.16±0.04 | 0.90±0.18 | 0.28±0.07 |

The biodistribution of biotin 2 in rat tissues is shown in Table 3. Five minutes after injection, without avidin, accumulation of biotin 2 in liver, kidney, and adrenal was greater than blood (P<0.001), heart (P<0.001), spleen (P<0.001), muscle (P<0.001), and bone (P<0.001). At 60 min. accumulation of biotin 2 in bone was greater than all other organs (P<0.001) and accumulation in adrenal and lung was greater than kidney (P<0.05), liver (P<0.02), normal muscle (P<0.001 ) and blood (P<0.001). The infected/normal tissue ratio was 0.58±0.07 indicating more binding to normal muscle than to the infection (Table 4). At 5 min. with avidin pretreatment, there was a decrease in accumulation of 2 in adrenal (P<0.01 ), kidney (P<0.001), spleen (P<0.001), and heart (P<0.05) compared to untreated rats. At 60 min. there was greater accumulation in blood (P<0.05), heart (P<0.05), spleen (P<0.001), kidney (P<0.01), and normal muscle (P<0.05) relative to untreated rats. Avidin pretreatment did not change the infected/normal tissue ratio.

**Table 3.**

| **Tissue distribution of** ^{**18**}**F-labeled biotin 2 in *E*. *coli* infected rats.** | | | | |
|---|---|---|---|---|
| Tissue | Without Avidin 5 Min. | 60 Min. | With Avidin 5 Min. | 60 Min. |
| Blood | 0.88±0.07 | 0.30±0.02 | 0.85±0.04 | 0.25±0.03 |
| Heart | 0.80±0.06 | 0.25±0.03 | 0.96±0.10 | 0.20±0.03 |
| Lung | 1.38±0.21 | 0.58±0.10 | 1.59±0.16 | 0.55±0.12 |
| Liver | 1.63±0.27 | 0.42±0.03 | 1.91±0.22 | 0.55±0.13 |
| Spleen | 1.70±0.68 | 0.26±0.01 | 0.99±0.10 | 0.51±0.09 |
| Kidney | 1.62±0.18 | 0.57±0.04 | 2.70±0.48 | 1.39±0.21 |
| Adrenal | 1.56±0.14 | 0.92±0.21 | 2.00±0.24 | 0.72±0.12 |
| Teste | 0.59±0.08 | 0.20±0.03 | 0.74±0.11 | 0.15±0.02 |
| Norm mus | 0.62±0.09 | 0.35±0.09 | 0.69±0.10 | 0.28±0.04 |
| Inf mus | 0.49±0.05 | 0.20±0.02 | 0.44±0.05 | 0.18±0.02 |
| bone | 0.73±0.09 | 1.17±0.21 | 0.86±0.12 | 1.10±0.20 |

**Table 4.**

| **Infected to normal tissue ratios of** ^{**18**}**F-labeled biotin 2 in *E. coli* infected rats.** | | | | |
|---|---|---|---|---|
| Tissue | Without Avidin 5 Min. | 60 Min. | With Avidin 5 Min. | 60 Min. |
| Blood | 0.56±0.04 | 0.68±0.06 | 0.51±0.07 | 0.71±0.05 |
| Heart | 0.61±0.03 | 0.81±0.05 | 0.46±0.03 | 0.91±0.05 |
| Lung | 0.36±0.05 | 0.43±0.20 | 0.28±0.02 | 0.33±0.06 |
| Liver | 0.31±0.04 | 0.48±0.33 | 0.23±0.02 | 0.33±0.05 |
| Spleen | 0.70±0.04 | 0.78±0.08 | 0.44±0.05 | 0.35±0.04 |
| Kidney | 0.30±0.02 | 0.35±0.03 | 0.16±0.02 | 0.13±0.02 |
| Adrenal | 0.31±0.02 | 0.23±0.05 | 0.22±0.03 | 0.214±0.03 |
| Teste | 0.84±0.10 | 1.04±0.11 | 0.59±0.03 | 1.15±0.06 |
| Norm mus | 0.80±0.12 | 0.58±0.07 | 0.65±0.10 | 0.64±0.07 |
| bone | 0.67±0.05 | 0.17±0.03 | 0.51±0.03 | 0.17±0.03 |

**Table 5.**

| **Comparison of Infected thigh to normal tissue ratios.** | | | | |
|---|---|---|---|---|
| Tissue | ¹¹¹In-IgG* | ¹¹¹In-Streptavidin* | Streptavidin/ ¹¹¹In-Biotin* | ¹⁸F-biotin1 |
| *normal muscle* | 4.5 | 4.4 | 13.0 | 6.1 |
| Blood | 0.3 | 0.8 | 6.2 | 3.1 |
| Liver | 1.1 | 1.2 | 9.3 | 3.5 |
| Kidney | 0.8 | 0.3 | 0.8 | 2.0 |

| | | | | |
|---|---|---|---|---|
| *As reported in Rusckowski M, Fritz B, Hnatowich DJ. Localization of infection using streptavidin and biotin: an alternative to nonspecific polyclonal immunoglobulin. *J. Nucl. Med. 33:* 1810-1815 (1992). | | | | |

**Table 6.**

| **Comparison of E. coli infected thigh to normal thigh ratios for various radiopharmaceuticals.** | | | |
|---|---|---|---|
| Agents | Infected/normal muscle ratio | Time | Reference |
| ⁶⁷Ga-citrate | 2.6±0.6 | 4 | *1 |
| ^{99m}Tc-HSA | 4.1±0.6 | 4 | *1 |
| ¹²⁵I-TNT-1 | 3.3±0.5 | 4 | *1 |
| ^{99m}Tc-TNT-1 | 3.4±0.08 | 4 | *1 |
| ^{99m}Tc.IgG | 3.0±1.1 | 4 | *1 |
| ¹¹¹In-IgG | 4.5 | 6 | *2 |
| ¹¹¹In-Streptavidin | 4.4 | 6 | *2 |
| Streptavidin + ¹¹¹In-Biotin | 13 | 3 | *2 |
| ¹¹¹In-Biotin | minimal | 3 | *2 |
| ¹⁸F-Biotin 1 | 6.1±1.1 | | |

| | | | |
|---|---|---|---|
| All values are reported for E. *coli* infection in right thigh. TNT = antinucleus antibody * 1 Hnatowich DJ et al., (1992) *J. Nucl. Med. 33*:934-935; *2 Rusckowski, M et al., (1992) *J. Nucl. Med. 33*:1810-1815. | | | |

### Example 4: Biotin Compounds for Treating Malaria and Tuberculosis

Antisense oligonucleotides to genes involved in the growth (e.g. aspartate semialdehyde dehydrogenase (asu) or aspartakinase (ask) or cell wall or mycolic acid synthesis of *Mycobacterium tuberculosis* and *Plasmodium falciparum* can be conjugated to a biotin compound as described above and administered to a subject to prevent or treat tuberculosis or malaria.

Those skilled in the art will recognize, or be able to ascertain using no more than routine experimentation, many equivalents of the specific embodiments described herein.

## Claims

1. Use of a biotin compound in the manufacture of a medicament or diagnostic material for use in a method of therapy or diagnosis involving targeting of said compound to a site in a patient without pre-administration of avidin or streptavidin, wherein the biotin compound is biotin (hexahydro-2-oxo-1H-thieno[3,4-d]-imidazoline-4-valeric acid); or a biotin amide analogue, having the following structural formula: 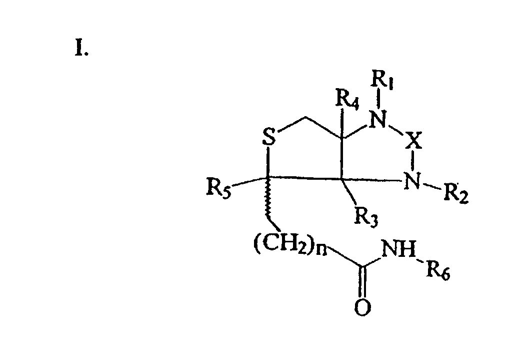 wherein,
n= 2-10;
R₁-R₅= H, acyl, alkyl, alkylene, alkenylene, alkynylene, alkenyl or alkynyl groups;
R₆= H, acyl, alkyl, alkylene, alkenylene, alkynylene, alkenyl or alkynyl groups; or a conjugated therapeutic agent or imaging agent; and
X= C=O, S=O or C=NH,

2. Use as specified in claim 1, wherein the biotin compound is biotin conjugated to an imaging or therapeutic agent.

3. Use as specified in claim 1 or claim 2, wherein said biotin compound is a biotin amide analogue conjugated to an imaging or therapeutic agent.

4. Use as specified in any preceding claim, wherein the biotin compound becomes localised at the site of infection within 1 hour after being administered.

5. Use as specified in any preceding claim, wherein the biotin compound becomes localised at the site of infection at a target to non-target ratio of at least 5:1.

6. Use as specified in any preceding claim, wherein the biotin compound comprises a therapeutic agent.

7. Use as specified in claim 6, wherein the therapeutic agent comprises a drug, toxin, radionuclide, hormone antagonist, heavy metal complex, oligonucleotide, chemotherapeutic nucleotide, peptide, non-specific (non-antibody) protein, boron containing compound or an enediyne.

8. Use as specified in claim 7, wherein the radionuclide is selected from: I-131, Re-186, Re-188, Y-90, Bi-212, At-211, Sr-89, Ho-166, Sm-153, Cu-67 or Cu-64.

9. Use as specified in any of claims 1 to 5, wherein the biotin compound comprises an imaging agent.

10. Use as specified in claim 9, wherein the imaging agent comprises: In-111, Tc-99m, I-123, I-125, F-18, Ga-67, Ga-68, a paramagnetic atom or a contrast agent.

11. Use as specified in any of claims 1 to 10, wherein said site comprises a site of a bacterial, viral or fungal infection and said medicament or diagnostic material is for the therapy or diagnosis of said infection.

12. Use as specified in claim 1 or claim 2, wherein the site comprises a tumour of the brain, lung, ovary, prostate, breast or colon and said medicament or diagnostic material is for the therapy or diagnosis of said tumour.

## Patentansprüche

1. Verwendung einer Biotinverbindung bei der Herstellung eines Medikaments oder Diagnostikums zum Einsatz bei einer Therapie- oder Diagnosemethode, bei der mit dieser Verbindung ohne vorherige Verabreichung von Avidin oder Streptavidin auf einen Krankheitsherd bei einem Patienten gezielt wird, **dadurch gekennzeichnet, dass** es sich bei der Biotinverbindung um Biotin (Hexahydro-2-oxo-1H-thieno[3,4-d]-imidazolin-4-Valeriansäure) oder eine Biotinamidanalogverbindung mit folgender Strukturformel handelt: 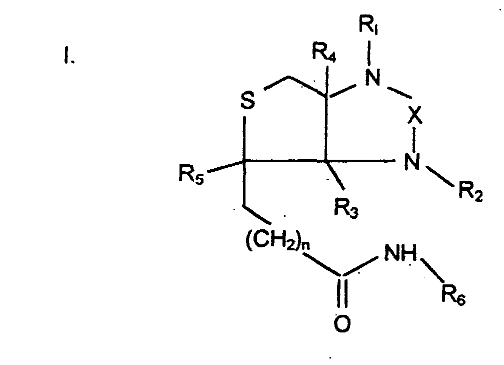 In dieser Formel bedeuten:
n = 2-10,
R₁-R₅ = H, Acyl-, Alkyl-, Alkylen-, Alkenylen-, Alkynylen-, Alkenyl- oder Alkynylgruppen
R₆ = H, Acyl-, Alkyl-, Alkylen-, Alkenylen-, Alkynylen-, Alkenyl- oder Alkynylgruppen oder ein konjugiertes Therapeutikum oder ein Bildgebungsmittel, und
X = C=O, S=O oder C=NH

2. Verwendung nach Anspruch 1, wobei die Biotinverbindung an ein Bildgebungsmittel oder ein Therapeutikum konjugiertes Biotin ist.

3. Verwendung nach Anspruch 1 oder 2, wobei die Biotinverbindung eine an ein Bildgebungsmittel oder ein Therapeutikum konjugierte Biotinamidanalogverbindung ist.

4. Verwendung nach den vorstehenden Ansprüche, wobei die Lokalisierung der Biotinverbindung am Infektionsherd innerhalb einer Stunde nach der Verabreichung eintritt.

5. Verwendung nach den vorstehenden Ansprüchen, wobei die Lokalisierung der Biotinverbindung am Infektionsherd ein Ziel-/Nicht-Ziel-Verhältnis von mindestens 5:1 aufweist.

6. Verwendung nach den vorstehenden Ansprüchen, wobei die Biotinverbindung ein Therapeutikum beinhaltet.

7. Verwendung nach Anspruch 6, wobei das Therapeutikum eine Verbindung, die ein Arzneimittel, ein Toxin, ein Radionuklid, einen Hormonantagonisten, einen Schwermetallkomplex, ein Oligonukleotid, ein chemotherapeutisches Nukleotid, ein Peptid, ein unspezifisches (Antikörper-) Protein oder Bor enthält, oder ein Enediyn beinhaltet.

8. Verwendung nach Anspruch 7, wobei das Radionuklid aus folgender Gruppe ausgewählt wird: I-131, Re-186, Re-188, Y-90, Bi-212, At-211, Sr-89, Ho-166, Sm-153, Cu-67 oder Cu-64.

9. Verwendung nach den Ansprüchen 1 bis 5, wobei die Biotinverbindung ein Bildgebungsmittel umfasst.

10. Verwendung nach Anspruch 9, wobei das Bildgebungsmittel In-111, Tc-99m, 1-123, I-125, F-18, Ga-67, Ga-68, ein paramagnetisches Atom oder ein Kontrastmittel umfasst.

11. Verwendung nach den Ansprüchen 1 bis 10, wobei der Krankheitsherd einen Herd einer bakteriellen, viralen oder Pilzinfektion einschließt und das Medikament oder Diagnostikum zur Therapie oder Diagnose dieser Infektion dient.

12. Verwendung nach Anspruch 1 oder 2, wobei der Krankheitsherd einen Tumor des Gehims, der Lunge, der Prostata, der Brust oder des Dickdarms einschließt und das Medikament oder Diagnostikum zur Therapie oder Diagnose dieses Tumors dient.

## Revendications

1. Utilisation d'un composé biotine dans la fabrication d'un médicament ou d'une substance diagnostique destiné à être utilisé dans une méthode de thérapie ou de diagnostic mettant en jeu le ciblage dudit composé à un site chez le patient sans administration préalable d'avidine ou de streptavidine, dans laquelle le composé de biotine est la biotine (acide hexahydro-2-oxo-1H-thiéno[3,4-d]-imidazoline-4-valérique); ou un analogue amide de biotine de formule structurelle suivante : 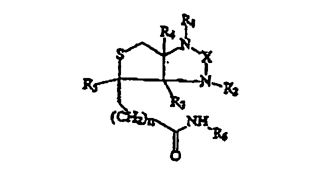 dans laquelle
n=2-10;
R₁ - R₅ = H, les groupes acyle, alkyle, alkylène, alcénylène, alcynylène, alcényle ou alcynyle ;
R₆ = H, les groupes acyle, alkyle, alkylène, alcénylène, alcynylène, alcényle ou alcynyle ; ou un agent thérapeutique ou un agent d'imagerie conjugué ; et
X= C=O, S= O ou C = NH.

2. Utilisation selon la revendication 1, dans laquelle le composé biotine est la biotine conjuguée à un agent d'imagerie ou thérapeutique.

3. Utilisation selon la revendication 1 ou la revendication 2, dans laquelle ledit composé biotine est un analogue amide de biotine conjugué à un agent d'imagerie ou thérapeutique.

4. Utilisation selon l'une quelconque des revendications précédentes, dans laquelle le composé biotine est localisé au site d'infection en 1 heure après avoir été administré.

5. Utilisation selon l'une quelconque des revendications précédentes, dans laquelle le composé biotine est localisé au site d'infection en un rapport cible/non cible d'au moins 5:1.

6. Utilisation selon l'une quelconque des revendications précédentes, dans laquelle le composé biotine comprend un agent thérapeutique.

7. Utilisation selon la revendication 6, dans laquelle l'agent thérapeutique comprend un médicament, une toxine, un radionucléide, un antagoniste hormonal, un complexe de métal lourd, un oligonucléotide, un nucléotide chimiothérapeutique, un peptide, une protéine (non anticorps) non spécifique, un composé contenant du bore ou un ène-diyne.

8. Utilisation selon la revendication 7, dans laquelle le radionucléide est choisi parmi : I-131, Re-186, Re-188, Y-90, Bi-212, At-211, Sr-89, Ho-166, Sm-153, Cu-67 ou Cu-64.

9. Utilisation selon l'une quelconque des revendications 1 à 5, dans laquelle le composé biotine comprend un agent d'imagerie.

10. Utilisation selon la revendication 9, dans laquelle l'agent d'imagerie comprend: In-111, Tc-99m, I-123, I-125, F-18, Ga-67, Ga-68, un atome paramagnétique ou un agent de contraste.

11. Utilisation selon l'une quelconque des revendications 1 à 10, dans laquelle ledit site comprend un site d'une infection bactérienne, virale ou fongique et ledit médicament ou ladite substance diagnostique sert à la thérapie ou au diagnostic de ladite infection.

12. Utilisation selon la revendication 1 ou la revendication 2, dans laquelle le site comprend une tumeur du cerveau, du poumon, de l'ovaire, de la prostate, du sein ou du côlon et ledit médicament ou ladite substance diagnostique sert à la thérapie ou au-diagnostic de ladite tumeur.
